Europäisches Patentamt

European Patent Office

Office européen des brevets

(11) Veröffentlichungsnummer: **0 286 830**
**A2**

# EUROPÄISCHE PATENTANMELDUNG

(21) Anmeldenummer: **88103665.1**

(22) Anmeldetag: **09.03.88**

(51) Int. Cl.4: **C07K 3/02**

(30) Priorität: **13.03.87 DE 3708244**

(43) Veröffentlichungstag der Anmeldung:
**19.10.88 Patentblatt 88/42**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Löbermann, Hartmut, Dr.**
**Mooswaldstrasse 7B**
**D-7801 Schallstadt(DE)**
Erfinder: **Bornmann, Christiane**
**Steinweg 32**
**D-3573 Gemünden (Wohra)(DE)**

(74) Vertreter: **Meyer-Dulheuer, Karl-Hermann, Dr.**
**et al**
**HOECHST Aktiengesellschaft Zentrale**
**Patentabteilung Postfach 80 03 20**
**D-6230 Frankfurt/Main 80(DE)**

(54) **Verfahren zur Extraktion von Protein PP4 aus Geweben und die Verwendung von Citronensäure für diesen Zweck.**

(57) Es wird ein Verfahren zur Extraktion des Proteins $PP^4$ aus vorzugsweise zerkleinertem oder homogenisiertem Gewebe beschrieben, worin dieses Gewebe gegebenenfalls nach Waschung mit einer Lösung eines Neutralsalzes mit einer ein lösliches Salz der Citronensäure enthaltenden Pufferlösung gegebenenfalls unter Rühren in Kontakt gebracht und die das $PP_4$ enthaltende Lösung abgetrennt wird.

EP 0 286 830 A2

## Verfahren zur Extraktion von Protein PP₄ aus Geweben und die Verwendung von Citronensäure für diesen Zweck

Die Erfindung betrifft ein Verfahren zur Extraktion des Gewebeproteins $PP_4$ aus Geweben.

Das Gewebeprotein $PP_4$ ist in DE 33 15 000 A 1 (USP 4,507,229) mit folgenden Parametern beschrieben:

- einer elektrophoretischen Beweglichkeit im Bereich zwischen der von $alpha_1$ und $alpha_2$ Globulinen;
- einem isoelektrischen Punkt von 4,85 ± 0,15;
- einem Sedimentationskoeffizienten $s_{20,w}^0$ von 3,3 ± 0,2S;
- einem im natriumdodecylsulfat (SDS)-haltigen Polyacrylamidgel bestimmtes Molekulargewicht von 35.000 ± 5 000;
- einem Extinktionskoeffizienten $E_{1cm}^{1\%}$ (280 nm) von 5,9 ± 0,6;
- einem Kohlenhydratanteil von 2,4 ± 0,94 % (g/100 g) (Mannose 0,3 ± 0,2 %, Galactose 0,4 ± 0,2 % Xylose 0,1 ± 0,04 %, Glukose 0,2 ± 0,1 %, Glukosamin 1,0 ± 0,2 %, Neuraminsäure 0,4 ± 0,2 %) und
- der folgenden Aminosäurenzusammensetzung:

| Aminosäure | Reste pro 100 Reste (Mol %) | Variations-koeffizient |
|---|---|---|
| Lysin | 6,95 | 1,14 |
| Histidin | 0,97 | 17,4 |
| Arginin | 5,44 | 1,77 |
| Asparaginsäure | 11,41 | 1,68 |
| Threonin | 6,78 | 2,40 |
| Serin | 6,21 | 2,26 |
| Glutaminsäure | 12,25 | 0,43 |
| Prolin | 1,96 | 6,20 |
| Glycin | 6,68 | 3,83 |
| Alanin | 7,92 | 1,67 |
| Cystin 1/2 | 0,77 | 19,5 |
| Valin | 5,34 | 3,80 |
| Methionin | 1,98 | 6,00 |
| Isoleucin | 5,21 | 2,23 |
| Leucin | 11,50 | 0,45 |
| Tyrosin | 3,55 | 4,21 |
| Phenylalanin | 4,07 | 3,77 |
| Tryptophan | 0,93 | 23,9 |

Da dieses Protein blutgerinnungshemmende Eigenschaften aufweist (DE 36 43 182) und von therapeutischem Interesse ist, ist eine einfache und möglichst quantitative Extraktion von $PP_4$ aus Geweben wie Plazenta von großer Bedeutung. Das bisherige Extraktionsverfahren (Arch. Gynaecol. (1985) 236, 225-233) benötigt mindestens zwei Extraktionsschritte, beispielsweise mit [R]Triton X-100 und danach mit Kaliumrhodanid, um eine möglichst vollständige Extraktion zu erzielen.

Aufgabe der Erfindung ist daher die Entwicklung eines Verfahrens zur Extraktion von $PP_4$ aus Geweben, das die Nachteile der Verfahren des Standes der Technik vermeidet und unter Einsatz nur einer Extraktionslösung zu einer möglichst vollständigen Extraktion von $PP_4$ führt. Außerdem sollte der Einsatz von Detergentien vermieden werden, da ihre Abtrennung die Aufarbeitung erschwert.

Es wurde überraschenderweise gefunden, daß eine Citrat enthaltende Lösung zur Extraktion von PP$_4$ aus Geweben verwendet werden kann.

Es wurde weiterhin überraschenderweise gefunden, daß bei Einhaltung eines geeigneten pH-Wertes und einer geeigneten Molarität von Citrat in der Lösung PP$_4$ in deutlich größerer Menge extrahiert werden kann, als dies mit den bisher bekannten Extraktionsmethoden möglich ist.

Gegenstand der Erfindung ist somit ein Verfahren zur Extraktion des Proteins PP$_4$ aus vorzugsweise zerkleinertem oder homogenisiertem Gewebe, dadurch gekennzeichnet, daß dieses Gewebe gegebenenfalls nach Waschung mit einer Lösung eines Neutralsalzes mit einer ein lösliches Salz der Citronensäure enthaltenden Pufferlösung gegebenenfalls unter Rühren in Kontakt gebracht und die das PP$_4$ enthaltende Lösung abgetrennt wird.

Der Extraktionsschritt kann gegebenenfalls wiederholt werden.

In einer bevorzugten Verfahrensweise wird das zerkleinerte und/oder homogenisierte Gewebe, beispielsweise reife, menschliche Plazenta, zunächst mit einer Lösung eines Neutralsalzes die auf einen pH-Wert von 5 - 9,5 eingestellt wird, enthaltend beispielsweise 0,01 - 1 mol/l NaCl, KCl oder LiCl und gegebenenfalls 0,005 - 0,5 mol/l Tris, Hepes oder Glycin mehrfach gewaschen und gegebenenfalls lyophilisiert. Das Gewebe wird dann mit einer Lösung eines Citronensäuresalzes, vorzugsweise 0,01 - 1,5 mol/l Na-, K-oder Li-Citrat, die gegebenenfalls andere Salze, beispielsweise 0,01 - 1,5 mol/l NaCl, KCl oder LiCl enthält, bei einem pH-Wert von 5 - 9,5 in Kontakt gebracht, vorzugsweise gemischt, und mindestens einige Minuten in Kontakt miteinander gelassen, vorzugsweise gerührt, und die Flüssigkeit abgetrennt, vorzugsweise durch Filtration oder Zentrifugation. Je 100 g feuchtem Gewebe werden vorzugsweise mindestens 200 ml citrat-haltige Lösung zugesetzt.

Es können zur Extraktion auch mehr als ein Salz der Citronensäure verwendet werden.

Besonders bevorzugt wird mit 0,05 - 0,2 mol/l NaCl, KCl oder LiCl gegebenenfalls enthaltend 0,01 - 0,2 mol/l Tris, Hepes oder Glycin, pH 6 - 8,5 gewaschen.

Extrahiert wird besonders bevorzugt mit einer 0,05 - 0,5 mol/l Na-, K-oder Li-Citrat-Lösung, pH 6 - 8,5.

Ganz besonders bevorzugt wird homogenisiertes Gewebe mit 0,1 - 0,2 mol/l NaCl gegebenenfalls enthaltend 0,02 - 0,1 mol/l Tris, Hepes oder Glycin, pH 7 - 8 gewaschen und mit einer Lösung, enthaltend 0,05 - 0,3 mol/l Na-, K-oder Li-Citrat bei einem pH von 7 - 8 extrahiert.

Die Extrakte, enthaltend PP$_4$, können nach bekannten Methoden, beispielsweise durch Ionenaustausch- oder Affinitätschromatographie und/oder Gelfiltration, gegebenenfalls nach Konzentrierung mittels Fällung oder Ultrafiltration oder auch Dialyse, weiter aufgearbeitet werden.

Dieses Verfahren zeichnet sich besonders dadurch aus, daß PP$_4$ mit nur einer Lösung extrahiert werden kann, daß auf die Verwendung von Detergentien verzichtet werden kann und PP$_4$ in deutlich größerer Menge extrahiert wird, als dies mit den bekannten Extraktionsmethoden möglich ist.

Die folgenden Beispiele erläutern die Erfindung.


Beispiel 1

500 g reife, menschliche Plazenta werden in einem Mixer zerkleinert und homogenisiert und 4mal mit je 2 l 0,9 g/100 ml NaCl-Lösung gewaschen. Anschließend wird das Gewebe 2 mal mit je 1 l 0,1 mol/l Tri-Na-Citrat-Lösung, pH 7,5, über Nacht extrahiert. Die vereinigten Extrakte (1750 ml) enthielten 40 mg PP$_4$.

Die Gehaltsbestimmung an PP$_4$ wurde mittels immunologischer Methoden, wie beispielsweise der Laurell-Elektrophorese mit monospezifischem Antiserum gegen PP$_4$ aus Kaninchen durchgeführt.


Beispiel 2

Die vorgewaschene Plazenta (500 g; Beispiel 1) wurde lyophilisiert. 10 g lyophilisiertes Gewebe ergeben mit Extraktionspuffer etwa 100 - 120 g feuchtes, gequollenes Gewebematerial. Die Extraktion wird zweimal über Nacht mit je 250 mmol/l Tri-K-Citrat, pH 7,5, durchgeführt. Die vereinigten Extrakte (1600 ml) enthielten 41 mg PP$_4$.

Beispiel 3

Ausbeuten von PP4 nach Extraktion von Plazentagewebe mit 3 mol/l KSCN, 2 g/100 ml $^R$Triton X-100 oder 100 mmol/l Na₃-Citrat pH 7,5:

| Extraktionslösung | Extrahierte PP₄-Menge in mg (600 g Plazenta) |
|---|---|
| 2 g/100 ml $^R$Triton X-100 (?) | 30 |
| 3 mol/1 KSCN | 17 |
| 0,1 mol/1 Na₃-Citrat, pH 7,5 | 41 |

**Ansprüche**

1. Verfahren zur Extraktion des Proteins PP4 aus vorzugsweise zerkleinertem oder homogenisiertem Gewebe, dadurch gekennzeichnet, daß dieses Gewebe gegebenenfalls nach Waschung mit einer Lösung eines Neutralsalzes mit einer ein lösliches Salz der Citronensäure enthaltenden Pufferlösung gegebenenfalls unter Rühren in Kontakt gebracht und die das PP4 enthaltende Lösung abgetrennt wird.

2. Verfahren nach Anspruch 1, dadurch gekennzeichnet, daß mit einer Lösung mit einem pH-Wert von 5 - 9,5 enthaltend 0,01 - 1 mol/l NaCl, KCl oder LiCl und gegebenenfalls 0,005 - 0,5 mol/l Tris, Hepes oder Glycin gewaschen wird.

3. Verfahren nach Anspruch 1, dadurch gekennzeichnet, daß mit einer Lösung enthaltend 0,01 - 1,5 mol/l Na-, K-oder Li-Citrat und gegebenenfalls 0,01 - 1,5 mol/l NaCl, KCl oder LiCl bei einem pH-Wert von 5 - 9,5 in Kontakt gebracht wird.

4. Verfahren nach Anspruch 1, dadurch gekennzeichnet, daß mit einer Lösung enthaltend 0,05 - 0,2 mol/l NaCl, KCl oder LiCl und 0,01 - 0,2 mol/l Tris, Hepes oder Glycin, pH 6 - 8,5 gewaschen wird.

5. Verfahren nach Anspruch 1, dadurch gekennzeichnet, daß das Gewebe mit einer 0,05 - 0,5 mol/l Na-, K-oder Li-Citrat-Lösung, pH 6 - 8,5 in Kontakt gebracht wird.

6. Verfahren nach Anspruch 1, dadurch gekennzeichnet, daß homogenisiertes Gewebe mit 0,1 - 0,2 mol/l NaCl gegebenenfalls enthaltend 0,02 - 0,1 mol/l Tris, Hepes oder Glycin, pH 7 - 8 gewaschen und mit einer Lösung, enthaltend 0,05 - 0,3 mol/l Na-, K-oder Li-Citrat bei einem pH von 7 - 8 in Kontakt gebracht wird. .

7. Verwendung von Citronensäure zur Extraktion des Gewebeproteins PP4 aus humanem Gewebe.